# EUROPÄISCHE PATENTSCHRIFT

(11) **EP 2 276 531 B1**
(45) Veröffentlichungstag und Bekanntmachung des Hinweises auf die Patenterteilung: **03.07.2013**
(21) Anmeldenummer: 09745677.6
(22) Anmeldetag: 06.05.2009
(51) Int. Cl.: A61M 15/00

(54) **SPENDER FÜR IN EINER GESONDERTEN VERPACKUNG ENTHALTENE, PULVERFÖRMIGE MASSEN**
DISPENSER FOR PULVERULENT COMPOUNDS CONTAINED IN A SEPARATE PACK
DISTRIBUTEUR POUR DES SUBSTANCES PULVÉRULENTES CONTENUES DANS UN EMBALLAGE SÉPARÉ

(30) Priorität: 13.05.2008 DE 102008023376
(43) Veröffentlichungstag der Anmeldung: 26.01.2011
(60) Teilanmeldung: 11150915.4
(73) Patentinhaber: Von Schuckmann, Alfred, 47627 Kevelaer (DE)
(72) Erfinder: Von Schuckmann, Alfred, 47627 Kevelaer (DE)
(74) Vertreter: Rieder, Hans-Joachim
(86) Internationale Anmeldenummer: PCT/EP2009/055468
(87) Internationale Veröffentlichungsnummer: WO 2009/138344

(56) Entgegenhaltungen:
- WO-A-2007/129127
- WO-A-2009/046072
- US-A1- 2002 108 611
- US-A1- 2005 252 511

## Beschreibung

Die Erfindung betrifft einen Spender für in einer gesonderten Verpackung enthaltene, pulverförmige Massen; insbesondere für in einer Blisterverpackung enthaltene Massen, gemäß Gattungsbegriff des Hauptanspruches.

Spender dieser Art sind durch die WO 2009/046072 A1 bekannt. Diese dienen vornehmlich der Inhalation medikamentöser, pulverförmiger Massen, aus sogenannten Blisterverpackungen. Bedeckt sind die mit der zu inhalierenden Masse befüllten Behälterteile durch eine auflaminierte Folie. Durch teilweises Abziehen der Behälterabdeckung wird der Inhalt freigelegt und kann ausgesaugt werden. Dazu ist der Spender als aus zwei Halbschalen gestaltetes Rohr ausgebildet, welches auf etwa mittiger Länge den kleinsten Querschnitt aufweist und dort den mit der Masse befüllten Behälterteil besitzt, dessen Abdeckung durch die Rohrwand nach außen gezogen werden kann, bis die Abdeckung den Schlitz möglichst verschließt. Die Positionierung des Abzugsbereiches insbesondere in die Zone der größten Durchström-Geschwindigkeit ist unbefriedigend, zumal jede Kontrollmöglichkeit fehlt für das richtige Maß des Abziehens der Behälterabdeckung.

Ein weitere Spender dieser Art ist in US-2005/251512 beschrieben.

Die Erfindung hat sich die Aufgabe gestellt, einen Spender der in Rede stehenden Art in baulich einfacher Weise so verbessert auszugestalten, dass eine funktionssichere Handhabung des Inhaliervorganges gegeben ist.

Diese Problematik ist zunächst und im Wesentlichen durch den Gegenstand des Anspruches 1 gelöst, wobei darauf abgestellt ist, dass das Abziehen der Behälterabdeckung nicht nur kontrollierbar ist, sondern auch außerhalb des Luft-Durchströmungsbereiches erfolgt. Der die Masse aus dem Behälterteil austragende Luftstrom wird im Zuge der Inhalation abseits der in die Behälterteilöffnungsstellung abgezogenen Behälterabdeckung eingesogen und strömt entsprechend nicht über dieselbe hinweg. Der das stets bestimmungsgemäß geöffnete Behälterteil ausräumende Luftstrom wird gezielt an der Behälterabdeckung vorbei zum Behälterteil geführt, wobei weiter die Behälterabdeckung genügend dicht ist, d.h. einem Ansaugen von Nebenluft ausreichend entgegengewirkt ist. Der Verschluss der Abziehöffnung allein durch die Behälterabdeckung braucht nicht zwingend absolut luftdicht zu sein.

Weitere Merkmale der Erfindung sind nachstehend und in der Figurenbeschreibung erläutert, alles unter anderem aus der Perspektive, dass es als vorteilhaft gefunden wurde, dass man den vollen Luftstrom nicht durch bzw. über den Behälterteil führt. So ist in einer Weiterbildung des Erfindungsgegenstandes auch vorgesehen, dass die eintretende Luft zunächst einen Sammelkanal durchsetzt, der sich in einen, unter Umgehung des Behälterteils zu einem Saugmund führenden Direktkanal und weiter in einen zum Behälterteil führenden Behälterkanal aufspaltet. Der Behälterkanal endet in bevorzugter Ausgestaltung vor Abziehen der Behälterabdeckung blind im Bereich des Behälterteils, welch letzterer nach Abzug der Behälterabdeckung Teil des Behälterkanals ist, womit die über den Behälterkanal geführte, abgeteilte Luftströmung das Behälterteil ausräumt. Bevorzugt taucht hierbei der abgeteilte Luftstrom durch das Behälterteil. Die Aufteilung der Luftströmungen nach dem Sammelkanal ist in einer Weiterbildung des Erfindungsgegenstandes gleichmäßig vorgenommen.

In vorteilhafter Ausgestaltung des Erfindungsgegenstandes kann auch vorgesehen sein, dass der Behälterkanal und der Direktkanal in der Wirbelkammer münden, so dass entsprechend die hierbei zusammengeführten Luftteilströme vereint den Saugmund verlassen. Bevorzugt ist diesbezüglich weiter, dass sich der Behälterkanal und der Direktkanal vor der Wirbelkammer vereinigen, so dass die Luftteilströme bereits zusammengeführt in die Wirbelkammer eintreten. Der Behälterkanal ist zufolge dieser Ausgestaltung in Art eines Bypass-Kanals zu dem Direktkanal ausgebildet, dies weiter bevorzugt in Strömungsrichtung vor der Wirbelkammer. In vorteilhafter Weise ist dabei vorgesehen, dass der Behälterkanal und/oder der Direktkanal in ein oberes Stockwerk der über zwei Stockwerke ausgebildeten Wirbelkammer treten. Es erfolgt entsprechend eine Durchströmung der doppelstöckigen Wirbelkammer von oben nach unten. Alternativ ist jedoch auch die Anströmung in die untere Etage und der Austritt aus der oberen Etage der Wirbelkammer möglich.

Die Markierungslinie kann hierbei Bestandteil des Spenders sein. Alternativ kann die Markierungslinie auch Teil des Behälterteils bzw. Teil der Behälterabdeckung sein. Jedenfalls dient die Markierungslinie als optische Hilfestellung für den Benutzer, um diesem die Minimal-Abzugsstellung der Behälterabdeckung anzuzeigen, in welcher Minimal-Abzugsstellung das Behälterteil sicher über die gesamte Öffnungsfläche freiliegt. So ist das ordnungsgemäße, vollständige Räumen des Behälterteils durch die durch den Behälterkanal geführte Luftströmung gesichert.

Nachstehend ist die Erfindung anhand der beigefügten Zeichnung, welche lediglich ein Ausführungsbeispiel darstellt, näher erläutert. Es zeigt:
- Fig. 1: einen Spender der in Rede stehenden Art in perspektivischer Darstellung, die Nichtbenutzungsstellung betreffend;
- Fig. 2: den Spender in Seitenansicht;
- Fig. 3: den Spender in einer weiteren perspektivischen Darstellung, nach Abnahme einer Saugmund-Schutzkappe;
- Fig. 4: die Draufsicht hierzu;

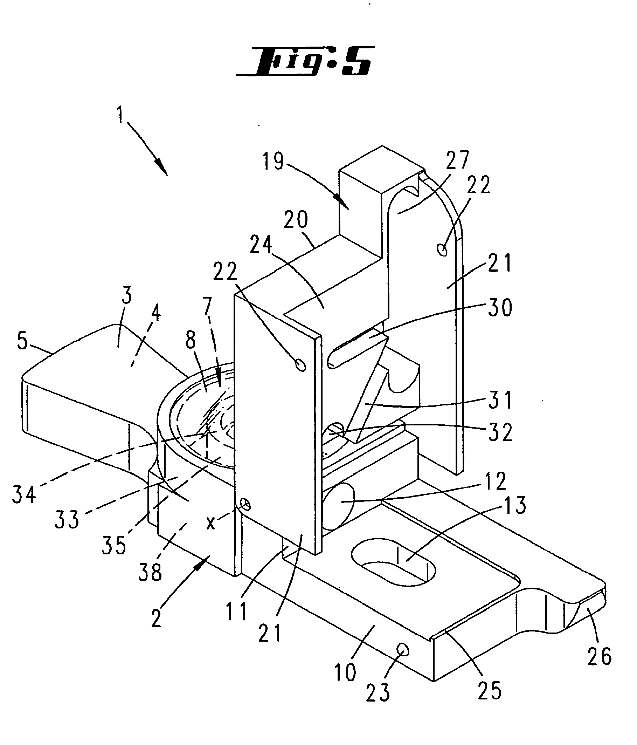

- Fig. 5: den Spender in perspektivischer Darstellung, eine Bereitschaftsstellung zur Bestückung mit einem Behälterteil betreffend;

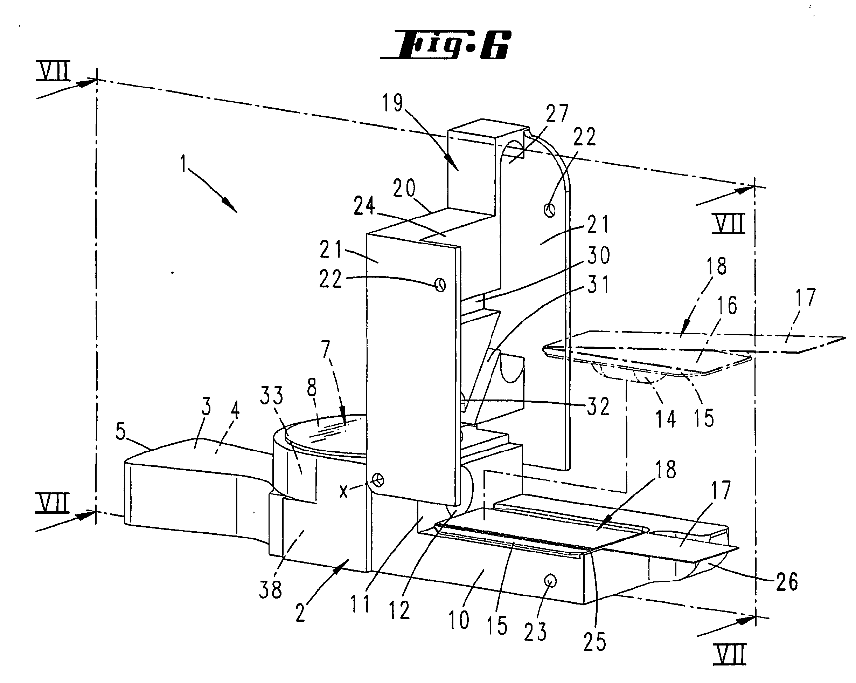

- Fig. 6: eine weitere perspektivische Darstellung des Spenders in der Bestückungs-Bereitschaftsstellung;
- Fig. 7: den Schnitt gemäß der Schnittebene VII-VII in Fig. 6;

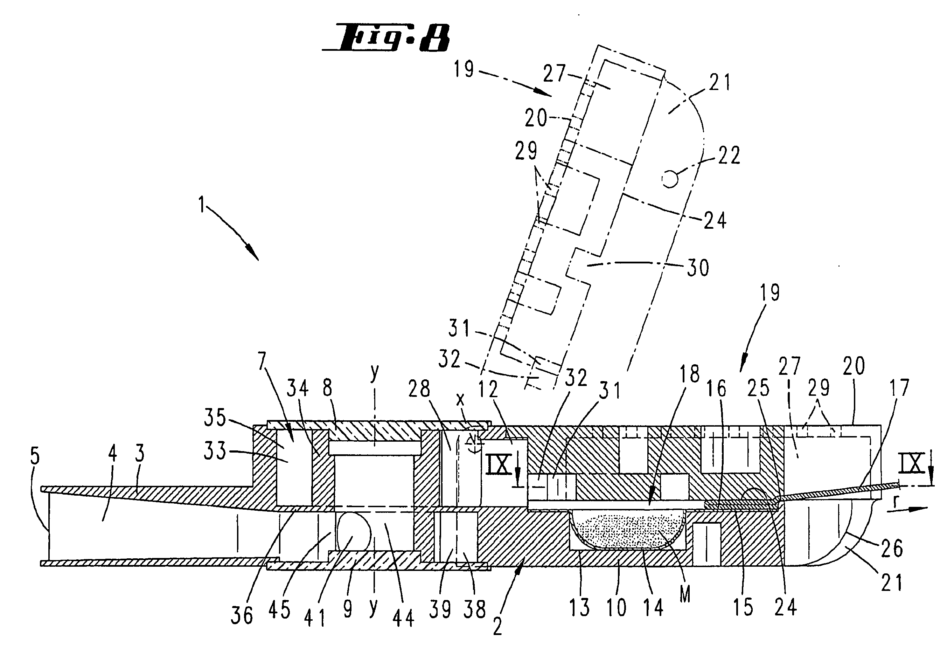

- Fig. 8: eine der Fig. 7 entsprechende Schnittdarstellung, jedoch die Spender-Bereitschaftsstellung betreffend;

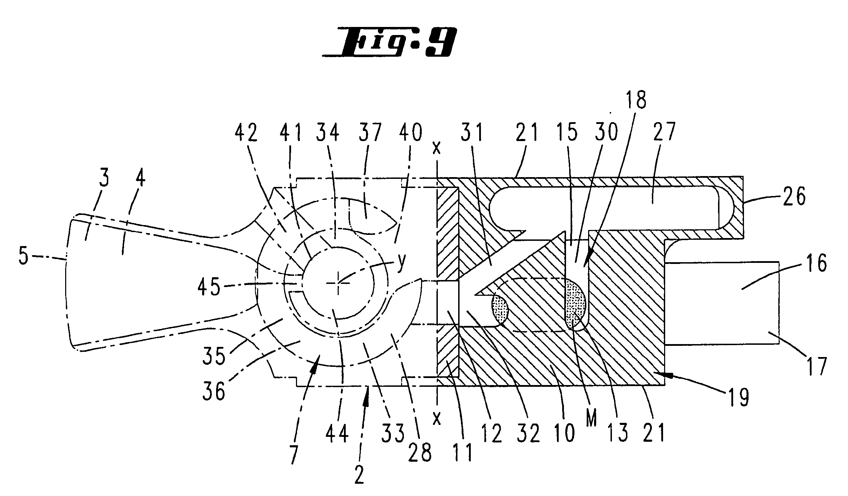

- Fig. 9: den Schnitt gemäß der Linie IX-IX in Fig. 8;

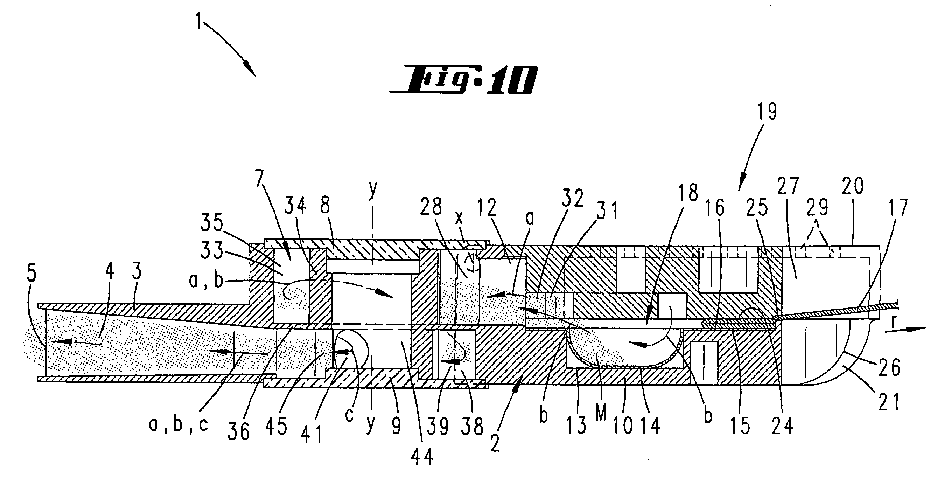

- Fig. 10: eine der Fig. 8 entsprechende Schnittdarstellung, jedoch im Zuge eines Inhaliervorganges;

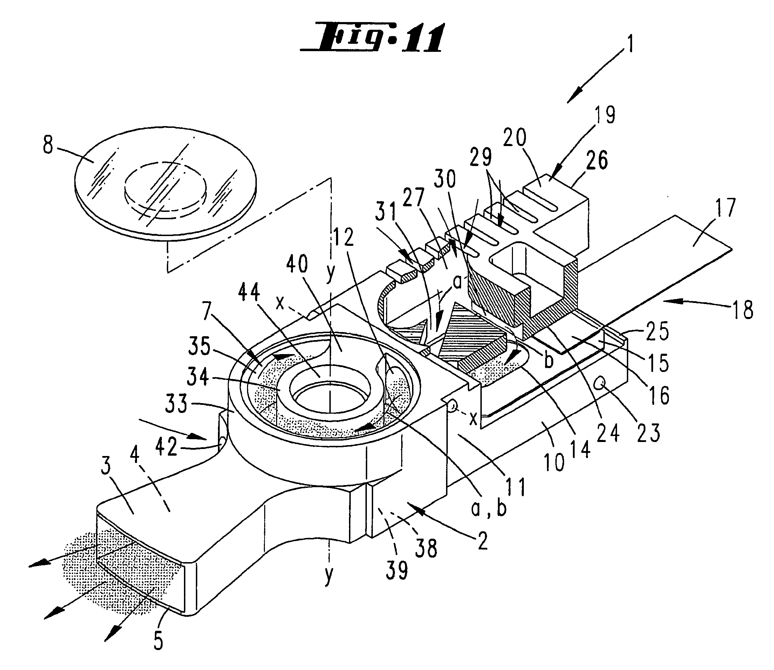

- Fig. 11: in teilweise explosionsartiger, partiell geschnittener Perspektivdarstellung den Spender im Zuge des Inhaliervorganges;

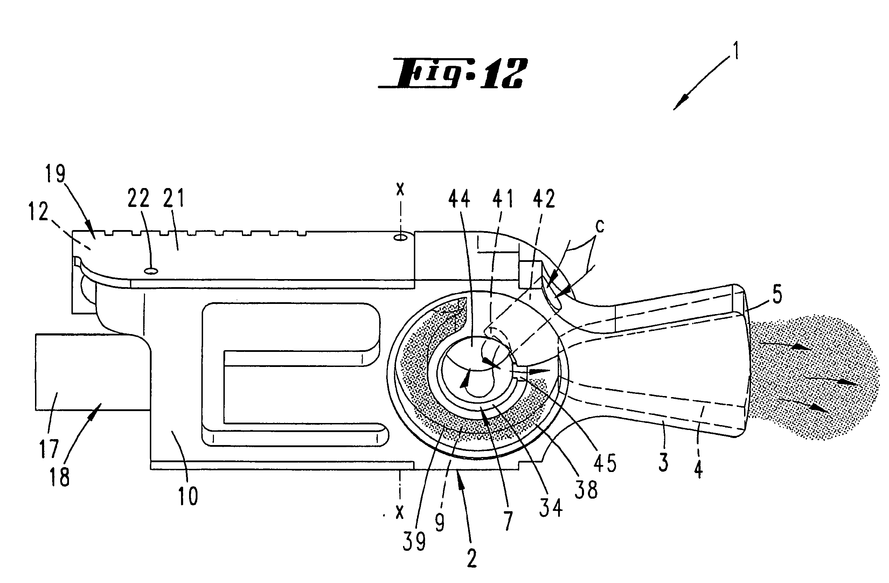

- Fig. 12: die perspektivische Unteransicht gegen den Spender während des Inhaliervorganges;
- Fig. 13: in perspektivischer Darstellung eine Vorratsbox zur Aufnahme des Spenders sowie einer Mehrzahl von dem Spender zuzuordnenden Behälterteilen;
- Fig. 14: den Schnitt gemäß der Linie XIV-XIV in Fig. 13.

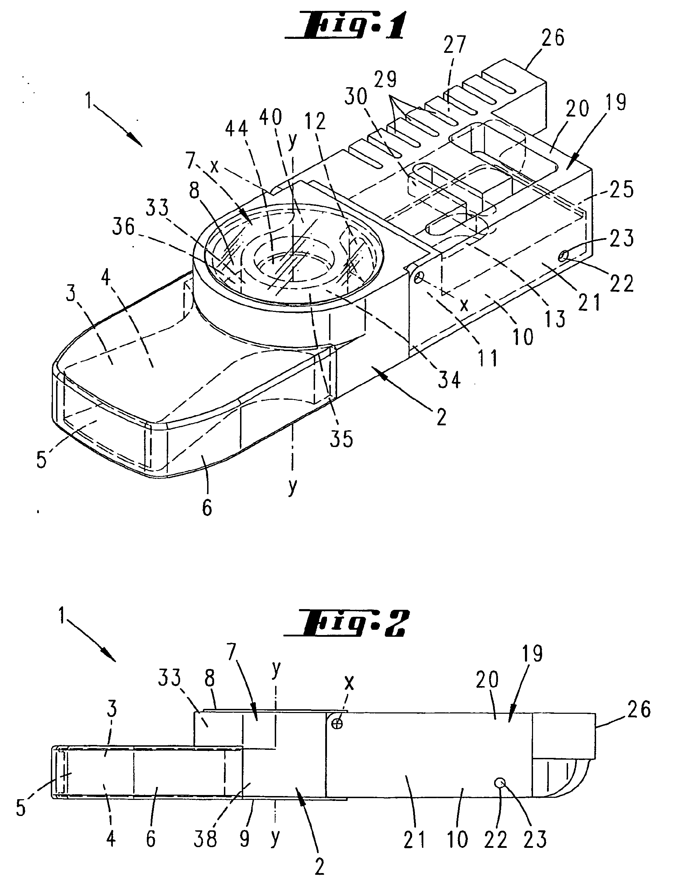

Dargestellt und beschrieben ist zunächst mit Bezug zu Fig. 1 ein Spender 1 in Art eines Inhalators, welcher als bequem mitführbares Taschengerät realisiert ist. Dieses weist ein im Wesentlichen langgestrecktes rechteckiges Gehäuse 2 auf, mit einem Längen-Breitenverhältnis von etwa 2 : 1 bis 2,5 : 1 und einer senkrecht zur Längserstreckung betrachteten Höhe, die etwa einem Viertel des Längserstreckungsmaßes entspricht. Die Teile des Spenders 1 sind als Kunststoff-Spritzteile realisiert.

In Verlängerung der Längserstreckung des Gehäuses 2 ragt von diesem ein Mundstück 3 ab. Der Übergang vom Gehäuse 2 zum Mundstück 3 ist bei insgesamt einteiliger Ausgestaltung mit Bezug auf die Breitenerstreckung des Gehäuses 2 tailliert.

Das Mundstück 3 ist in Längserstreckung durchsetzt von einem Saugkanal 4, der ausgangsseitig in einem Saugmund 5 endet.

Bei Nichtbenutzung des Spenders 1 ist das Mundstück 3 gemäß den Darstellungen in den Fig. 1 und 2 durch eine Verschlusskappe 6 überdeckbar. Diese ist die Breite des Gehäuses 2 aufnehmend im Bereich des taillenartigen Übergangs von Mundstück 3 zum Gehäuse 2 steckhalterbar.

Dem Übergangsbereich von Gehäuse 2 zum Mundstück 3 zugeordnet ist in dem Gehäuse 2 eine kreisrunde Wirbelkammer 7 ausgebildet. Diese erstreckt sich über zwei Stockwerke über die gesamte Höhe des Gehäuses 2, wobei die Kammerdecke 8 sowie der Kammerboden 9 durch kreisscheibenförmige Deckplatten gebildet sind, die steckhalterbar an dem Gehäuse 2 festgelegt sind. In dem dargestellten Ausführungsbeispiel sind Kammerdecke 8 und Kammerboden 9 transparent gestaltet und zur handhabungstechnisch einfachen Säuberung der Wirbelkammer 7 abnehmbar an dem Gehäuse 2 steckgehaltert.

Das dem Mundstück 3 abgewandte, sich an der Wirbelkammer 7 anschließende Gehäuseteil ist höhenmäßig abgestuft ausgebildet. Die verbleibende, gegenüber der Wirbelkammer-Decke 8 vertikal versetzte Oberfläche dieses Gehäuseteils 10 erstreckt sich etwa mit Bezug auf die Höhenerstreckung des Gehäuses 2 in einer Mittelebene.

Stirnseitig, d. h. der abgesetzten Ebene des Gehäuseteils 10 zugewandt mündet in der sich an der Wirbelkammer 7 gegenüberliegend zum Mundstück 3 anschließenden Stufe 11 ein in Längserstreckung des Gehäuses 2 ausgerichteter Luftkanal 12. Dieser weist einen der Stufenhöhe angepassten Durchmesser auf. Anderendig mündet dieser Luftkanal 12 in der Wirbelkammer 7.

In dem Gehäuseteil 10 ist eine zur ebenen Oberfläche hin sich öffnende Aufnahme 13 ausgebildet. Diese weist einen langlochartigen Grundriss auf und ist weiter mit Abstand zur Luftkanalmündung in der Stufe 11 in axialer Verlängerung des Luftkanals 12 ausgeformt. Die Kontur und Tiefe der Aufnahme 13 ist angepasst an die Kontur und Höhe eines aufzunehmenden Behälterteils 14.

Wie aus den weiteren Darstellungen zu erkennen, ist das Behälterteil 14 in Art einer Blisterverpackung gestaltet, wobei das Behälterteil 14 eine pulverförmige Masse M bevorratet. Hierzu ist das Behälterteil 14 wannenartig aus einem Kunststoffmaterial gefertigt, wobei weiter sich an dem umlaufenden Öffnungsrand des Behälterteils 14 ein ebenflächiger Stützabschnitt 15 anschließt. Insgesamt ist das Behälterteil 14 als Vertiefung aus dem Stützabschnitt 15 gebildet.

Die in dem Behälterteil 14 bevorratete Masse M ist in Nichtbenutzungsstellung versiegelt durch eine den Stützabschnitt 15 und das Behälterteil 14 vollflächig überdeckende Aluminiumfolie, die die Behälterabdeckung 16 darstellt. Diese Behälterabdeckung 16 ist zur Freigabe des Behälterteils 14 bzw. der darin bevorrateten Masse M abziehbar, wozu die Aluminiumfolie bzw. die Behälterabdeckung 16 ausgehend von einem quer zur Längserstreckung des Behälterteils 14 ausgerichteten Schmalbereich des Stützabschnittes 15 sich weiter in Gegenrichtung frei erstreckt und hierbei den das Behälterteil 14 und den Stützabschnitt 15 versiegelnden Bereich der Behälterabdeckung 16 frei aufliegend überdeckt. Das freie Ende der Behälterabdeckung 16 ragt frei über die dem Wendebereich der Behälterabdeckung 16 abgewandten Stirnrandkante des Stützabschnittes 15 aus, zur Ausbildung einer Abziehhandhabe 17.

Die in dem Gehäuseteil 10 ausgeformte Aufnahme 13 für das Behälterteil 14 ist in einem, gegenüber der Gehäuseteil-Oberfläche vertikal tiefer versetzten Bereich positioniert. Der Vertikalversatz entspricht im Wesentlichen der Materialstärke des sich aus Behälterteil 14, Behälterabdeckung 16 und Stützabschnitt 15 zusammensetzenden Blisters 18 außerhalb des Behälterteils 14. Weiter ist insbesondere die in Längserstreckung des Gehäuses 2 betrachtete Länge der Vertiefung angepasst an die in Längserstreckung des Behälterteiles 14 betrachtete Länge des Stützabschnittes 15. Die die Öffnung 13 umgebende Oberfläche der Vertiefung dient als Auflage des Stützabschnittes 15 bei eingelegtem Blister 18.

Dem Gehäuseteil 10 ist eine Öffnungsklappe 19 zugeordnet. Diese ist im Bereich der Stufe 11 um eine quer zur Längserstreckung des Gehäuse gerichtete Ackse x klappbar an dem Gehäuseteil 10 angelenkt. Die Öffnungsklappe 19 weist eine Klappendecke 20 auf. Beidseitig in Längserstreckung des Gehäuses 2 verlaufende Seitenwände 21 sind einstückig mit der Klappendecke 20 ausgebildet und flankieren in der Klappenschließstellung die zugeordneten Seitenflächen des feststehenden Gehäuseteils 10.

Die Öffnungsklappe 19 ist in der Klappenverschlussstellung rastgehaltert, wozu in den Seitenwänden 21 Rastvertiefungen 22 vorgesehen sind, in welche gehäuseteilseitige, linsenkopfartige Vorsprünge 23 eintauchen. Die so gebildete Rasthalterung ist durch den Anwender zum Öffnen der Klappe 19 leicht überwindbar.

Die Deckenunterseite 24 liegt in der Klappenverschlussstellung flächig auf der zugewandten Oberseite des Gehäuseteils 10 auf, so zumindest auf der, die Aufnahme 13 aufnehmenden Vertiefung umgebenden Planfläche. Bei eingelegtem Blister in die gehäuseteilseitige Vertriefung und damit einhergehendem Ausgleichen des Vertikalversatzes in dem Gehäuseteil 10 liegt entsprechend die Deckenunterseite 24 auf dem Blister 18, insbesondere auf der, die Abziehhandhabe 17 im freien Endbereich ausbildenden Behälterabdeckung 16. Entsprechend ist der eingelegte Blister 18 in Klappenverschlussstellung zum einen durch das annähernd formschlüssige Einliegen des Behälterteiles 14 in der Aufnahme 13 und zum weiteren ober- und unterseitig durch die Klappendecke und der zugeordneten Oberfläche der gehäuseteilseitigen Vertiefung gefasst, wobei weiter eine seitliche Abstützung des Blisters 18 über den Stützabschnitt 15 zum einen durch die Stufe 11 und die zugewandte Seitenwand 21 der Öffnungsklappe 19 und zum anderen durch die Randbegrenzung der Vertiefung gegeben ist.

Der Blister 18 ist derart in den Spender 1 einzulegen, dass die Umschlagkante der Behälterabdeckung 16 unter paralleler Ausrichtung zur Klappenachse x dem Luftkanal 12 zugewandt angeordnet ist und weiter die frei ausladende Abziehhandhabe 17 in entgegengesetzter Richtung über die Vertiefungsberandung hinaus aus dem Gehäuse 2 ragt, dies unter Durchsetzung einer entsprechend zwischen Gehäuseteil 10 und in Verschlussstellung verschwenkter Öffnungsklappe 19 belassenen, schlitzförmigen Abziehöffnung 25. Letztere ist in ihrer quer zur Längserstreckung des Gehäuses 2 betrachteten Breite angepasst an die Breite der Behälterabdeckung 16. Die vertikale Höhe der Abziehöffnung 25 entspricht im Wesentlichen der Materialstärke der Behälterabdeckung 16, so dass Letztere die Abziehöffnung 25 verschließt.

Eine korrekte Ausrichtung des einzuliegenden Blisters 18 in dem Spender 1 ist durch eine in Längserstreckung des Spenders 1 bzw. der Vertiefung in dem Gehäuseteil 10 vorgesehenen außermittigen Anordnung der Aufnahme 13. Der Blister 18 weist entsprechend in Längserstreckung an die außermittige Anordnung der Aufnahme 13 angepasste unterschiedliche Schenkellängen des Stützabschnittes 15 auf, so dass ein Einlegen des Blisters 18 ausschließlich in der vorbestimmten Ausrichtung erreicht werden kann.

Die Abziehöffnung 25 ist in einem gegenüber der Geräterückwand 26 in Richtung auf die Aufnahme 13 rückversetzten Bereich des Gehäuseteiles 10 bzw. der Öffnungsklappe 19 ausgeformt, so dass in der Bereitschaftsstellung, d. h. in der Klappenverschlussstellung bei eingelegtem Blister 18 die fahnenartig durch die Abziehöffnung 25 nach außen freiliegende Abziehhandhabe 17 einseitig durch den die Rückwand 26 aufweisenden Gehäuseteil-/Öffnungsklappen-Abschnitt flankiert ist.

Der über den die Abziehöffnung 25 mit ausformenden Abschnitt hinausragende und die Rückwand 26 tragende Bereich der Öffnungsklappe 19 ist mit einem sich in Längserstreckung des Gehäuses 2 bzw. der Öffnungsklappe 19 verlaufenden Sammelkanal 27 versehen. Dieser öffnet zunächst vollflächig zur Deckenunterseite 24 und ist auf einer Längsseite begrenzt durch die zugeordnete Seitenwand 21. Oberseitig ist der Sammelkanal 27 im Wesentlichen überdeckt durch die Klappendecke 20. Zum Anschluss des Sammelkanals 27 an die Umgebung ist die Klappendecke 20 mit parallel zur Klappachse x ausgerichteten, nutartigen Einlassöffnungen 29 durchsetzt, die entsprechend nach oben hin und weiter auch seitlich, die zugeordnete Seitenwand 21 durchsetzend öffnen. Es ist so eine einlassgitterartige Abdeckung des Sammelkanals 27 erreicht, wobei in dem dargestellten Ausführungsbeispiel acht nutartige Einlassöffnungen 29 gleichmäßig verteilt über die Länge des Sammelkanals 27 vorgesehen sind.

Quergerichtet zum Sammelkanal 27 geht ein stichkanalartiger Behälterkanal 30 aus. Dieser ist zur Deckenunterseite hin 24 wie auch der Sammelkanal 27 zunächst offen gestaltet. Den bodenseitigen Kanalabschluss sowohl des Sammelkanals 27 als auch des Behälterkanals 30 ist nach Verschwenken der Öffnungsklappe 19 in die Verschlussstellung durch die zugewandte Oberfläche des Gehäuseteiles 10 bzw. bei dem Behälterkanal 30 durch die zugewandte Oberfläche des Blisters 18 gegeben.

Mit Bezug auf die Klappenverschlussstellung erstreckt sich der Behälterkanal 30 bis in Überdeckung zu dem Behälterteil 14, dies weiter zugeordnet dem der Stufe 11 abgewandten Endbereich des eingelegten Behälterteils 14.

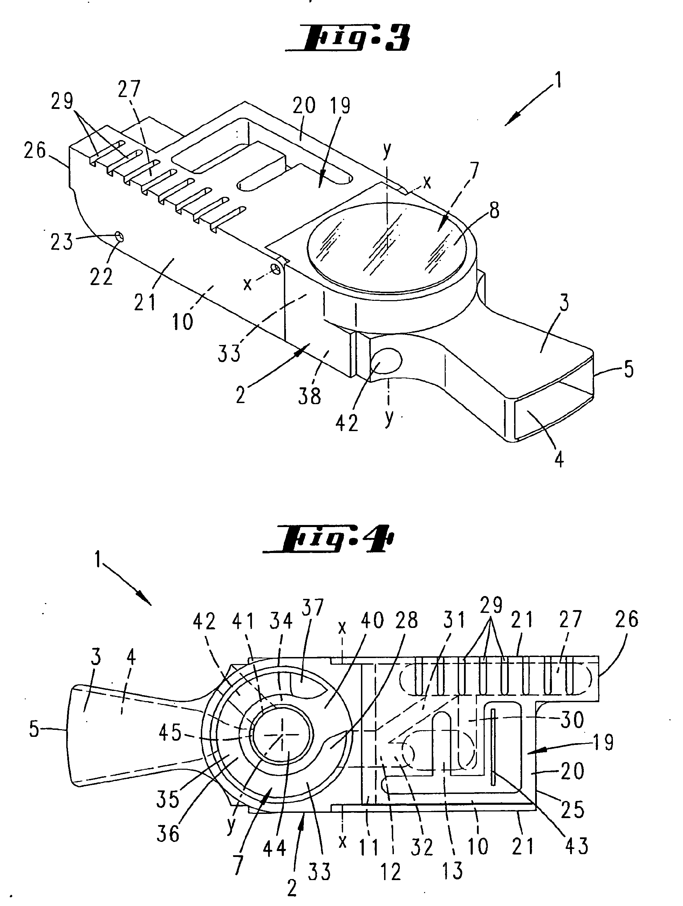

Darüber hinaus geht vom Wurzelbereich des Behälterkanals 30 in den Sammelkanal 27 ein gleichfalls in der Öffnungsklappe 19 ausgeformter, in der Klappenöffnungsstellung zur Deckenunterseite 24 gleichfalls offener Direktkanal 31 aus. Dieser erstreckt sich im Grundriss gemäß der Darstellung in Fig. 4 etwa einen Winkel von 30° zur Längserstreckung des Sammelkanals 27 einschließend in Richtung auf einen, in der Klappenschließstellung dem stufenseitigen Luftkanal 12 des Gehäuseteils 10 zugeordneten Vereinigungsabschnitt 32. Letzterer, wie auch der Direktkanal 31 sind bodenseitig in der Klappenverschlussstellung durch die zugeordnete Blisteroberfläche (Behälterabdeckung 16 bzw. Stützabschnitt 15) geschlossen.

Der klappenseitige, zur Deckenunterseite 24 hin sich öffnende Vereinigungsabschnitt 32 erstreckt sich in der Klappenverschlussstellung ausgehend von dem stufenseitig mündenden Luftkanal 12 bis in Überdeckung zu dem der Stufe 11 zugewandten Endabschnitt der Aufnahme 13 bzw. des in der Aufnahme 13 aufgenommenen Behälterteils 14.

Der gehäuseseitige, zum Gehäuseteil 10 hin öffnende Luftkanal 12 mündet andernends in einem Umlenkabschnitt 28 der Wirbelkammer 7, weiter in dem oberen Stockwerk 33 der Wirbelkammer 7, welches obere Stockwerk 33 sich im Wesentlichen oberhalb der Trennebene zwischen Gehäuseteil 10 und Öffnungsklappe 19 erstreckt.

Das obere Stockwerk 33 der Wirbelkammer 7 ist im Wesentlichen ringförmig gestaltet, wozu zentral eine sich über die gesamte Höhe des oberen Stockwerks 33 erstreckende Ringwandung 34 vorgesehen ist. Ausgehend vom Luftkanaleintritt erstreckt sich der Ringkanal 35 des oberen Stockwerks 33 etwa über einen Winkel von 270° (mit Bezug auf die Darstellung in Fig. 4 in Uhrzeigerrichtung) bevor der Ringraum 35 nach Durchsetzen einer den Stockwerksboden 36 öffnenden Durchbrechung 37 übergeht in einen im darunter angeordneten Stockwerk 38 gleichfalls ausgeformten Ringraum 39. Auch dieser ist radial innen begrenzt durch die im Wesentlichen über die gesamte vertikale Höhe der Wirbelkammer 7 durchlaufende Ringwandung 34.

Unmittelbar nachgeordnet der bodenseitigen Öffnung 37 ist im Bereich des oberen Stockwerks 33 eine den Ringraum in Umströmungsrichtung zum Eintritt des Luftkanals 12 verschließende Trennwand 40 gezogen, die radial von dem zugeordneten Ringwandungsabschnitt ausgeht.

Der Ringraum 39 des unteren Stockwerks 38 erstreckt sich ausgehend von der Öffnung 37 zum oberen Stockwerk 33 gleichfalls über etwa 270° bis zum Anschluss an den Saugkanal 4 des Mundstückes 3.

Es ist so eine im Wesentlichen helixförmige Luftführung in der Wirbelkammer 7 gegeben, dies unter Umlauf der durch die Wirbelkammer 7 gesogenen Luft um insgesamt 540°, dies weiter unter Durchsetzung zweier untereinander angeordneter Ebenen.

In den durch die Ringwandung 34 zentral in der Wirbelkammer 7 belassenen und durch die Ringwandung 34 im Wesentlichem zu den Ringräumen 35 und 39 abgeschirmten Zentralbereich der Wirbelkammer 7 mündet eine Außenluftöffnung 41, deren Außenluftkanal 42 radial zur Wirbelkammerachse y ausgerichtet ist. Das freie Ende des Außenluftkanals 42 mündet im taillenartigen Übergangsbereich vom Gehäuse 2 zum Mundstück 3, womit der Außenluftkanal 42 bzw. eine hierdurch definierte Ansaugrichtung durch den Kanal 42 unter Einschluss eines Winkels von etwa 45° zu einer Gehäuselängsachse annähernd entgegengerichtet ist zu der Haupt-Luftströmung des Spenders 1, welche Haupt-Luftströmung im Wesentlichen gleichgerichtet ist zur Längserstreckung des Gehäuses 2 bzw. zu einer Abziehrichtung r der Behälterabdeckung 16, nämlich weiter ausgehend im Wesentlichen von der Rückwand 26 des Gehäuses 2 bzw. der Öffnungsklappe 19 bis hin zum mundstückseitigen Saugmund 5.

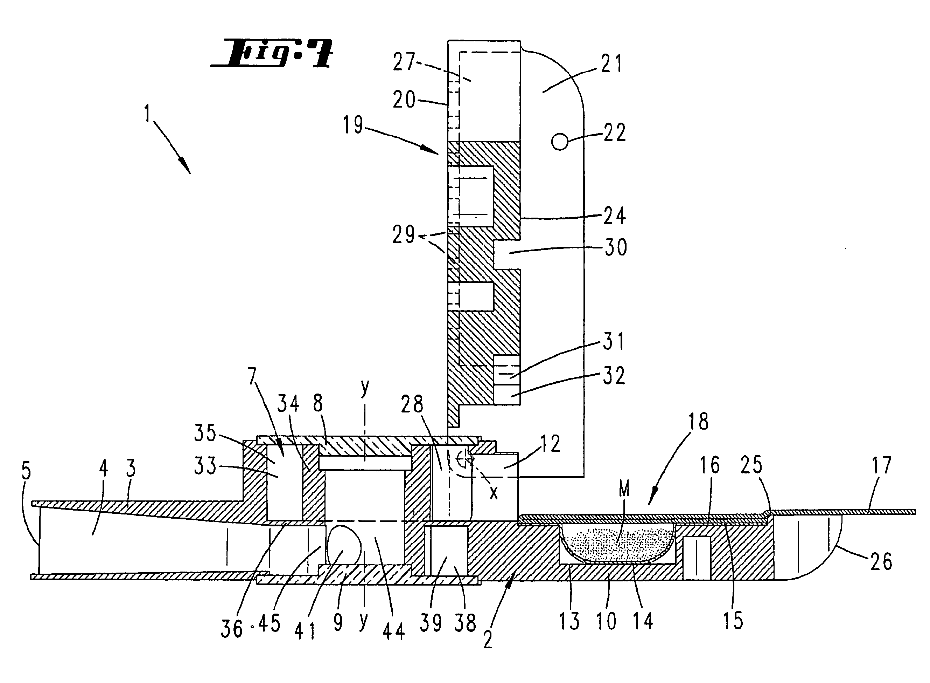

Zum Inhalieren einer Masse M wird zunächst nach Aufschwenken der Öffnungsklappe 19 in die Offenstellung gemäß Fig. 7 der Blister 18 eingelegt derart, dass das Behälterteil 14 in der Aufnahme 13 aufgenommen ist, dies unter Abstützung des blisterseitigen Stützabschnittes 15 auf der zugeordneten, vertieften Oberfläche des Gehäuseteiles 10. Die frei ausragende Abziehhandhabe 17 steht zur Bedienung frei über das Gehäuseende hinaus.

Nach Schließen der Öffnungsklappe 19 ist der Blister 18 in dem Gehäuse 2 gesichert. Es erfolgt hiernach das Öffnen des Behälterteiles 14 durch abrollendes Abziehen der Behälterabdeckung 16, wozu die durch die Abziehöffnung 25 hinausragende Abziehhandhabe 17 in Abziehrichtung r gezogen wird. Dieser Abziehvorgang ist in dem dargestellten Ausführungsbeispiel durch eine transparente Ausgestaltung der Öffnungsklappe 19 sichtbar. Die Öffnungsklappe 19 besitzt eine beispielsweise farbige Markierungslinie 43, bis zu welcher die Behälterabdeckung 16 gezogen werden muss. Entsprechend ist für den Benutzer eine Orientierung zum ordnungsgemäßen Gebrauch des Spenders 1 gegeben. Ein Abziehen der Behälterabdeckung 16 über die Sollstellung hinaus ist verhindert durch eine endseitige, nicht lösbare Verbindung von Behälterabdeckung 16 und Stützabschnitt 15.

Nach Abziehen der Behälterabdeckung 16 vom Behälterteil 14 liegt die Masse M im Gehäuse 2 frei. Das Behälterteil 14 ist bedingt durch die fehlende Abdeckung nunmehr strömungsmäßig einerends angeschlossen an den zum Sammelkanal 27 führenden Direktkanal 31 und andernends an den Vereinigungsabschnitt 32, so dass nunmehr der Behälterteil 14 den Direktkanal 31 mit der Vereinigungsabschnitt 32 strömungsmäßig verbindet und somit Teil des Kanals ist.

Der Spender 1 wird zur Inhalation flötenartig, bevorzugt mit Daumen und Zeigefinger gehalten. Durch Ansaugen über das Mundstück 3 tritt Luft durch die Einlassöffnungen 29 in den Sammelkanal 27 ein, wonach die eintretende Luft sich aufteilt zur Durchsetzung des Direktkanals 31 und zur Durchsetzung des Behälterkanals 30. Über diesen Behälterkanal 30 wird die in dem Behälterteil 14 bevorratete pulverförmige Masse M ausgetragen, wonach sich der massenversetzte Luftanteil b in dem Vereinigungsabschnitt 32 mit dem Luftanteil a durch den Direktkanal 31 vereinigt. Unter Durchsetzung der spiralförmigen, doppelstöckigen Wirbelkammer 7 erfolgt eine gleichmäßige Verteilung der Masse M im Luftstrom. Vor Austritt aus der Wirbelkammer 7 in den Saugkanal 4 des Mundstückes 3 tritt weitere Außenluft c über die Außenluftöffnung 41 bei. Die hierbei nebenluftartig angesogene Außenluft c tritt durch den Außenluftkanal 42 in den Zentralraum 44 der Wirbelkammer 7 und wird über eine zum unteren Ringraum 39 unmittelbar in Richtung auf den Saugkanal 4 gerichtete Durchströmöffnung 45 gesogen, zur Vereinigung mit dem massenbehafteten Luftstrom a, b aus dem Gehäuse 2 unmittelbar vor Übergang in den Saugkanal 4.

Im Zuge des Inhaliervorganges verschließt die freie Behälterabdeckungs-Fahne die Abziehöffnung 25, womit einem Ansaugen von Nebenluft entgegengewirkt ist.

Zufolge der beschriebenen Ausgestaltung erfolgt die Ansaugung bzw. die Luftführung innerhalb des Gehäuses 2 unter Umgehung der zur Inhalation in die Abziehstellung verlagerten Behälterabdeckung 16, die im Zuge der Inhalation zugleich die Abziehöffnung 25 verschließt. Der die Masse M aus dem Behälterteil 14 ausräumende Saugluftstrom ist irritationsfrei geführt.

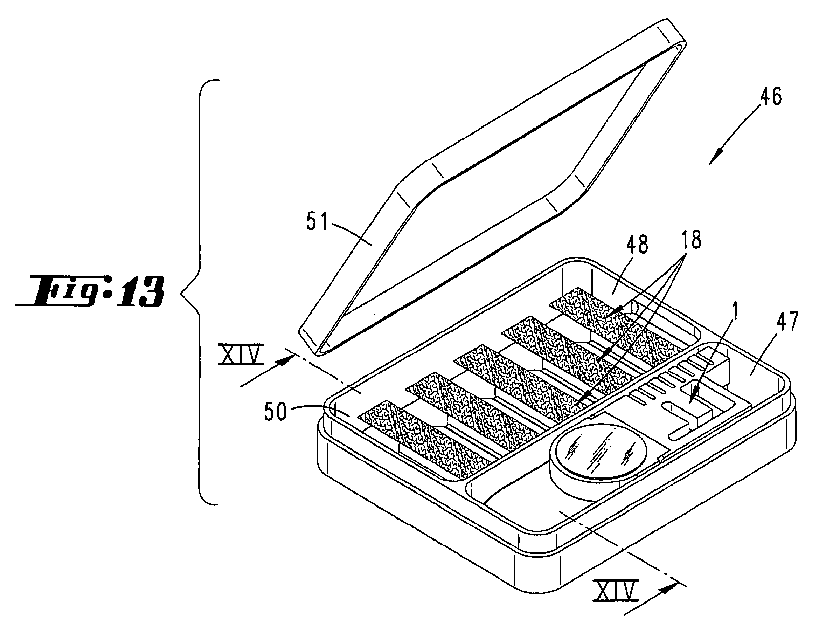

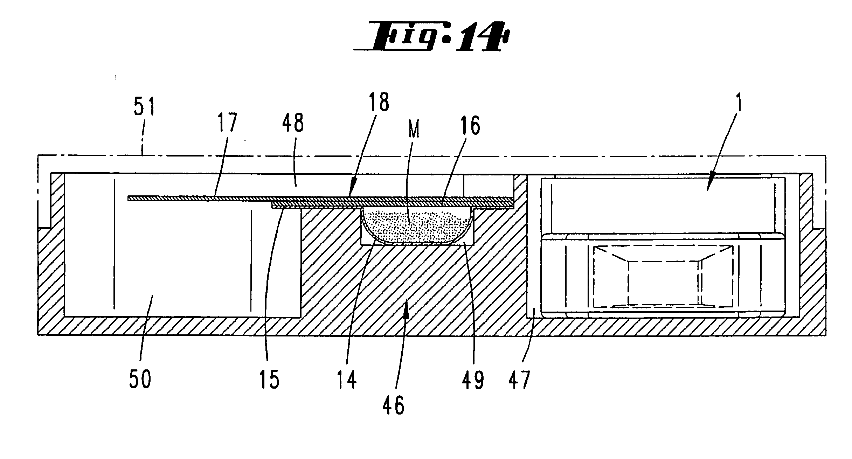

Wie in den Fig. 13 und 14 dargestellt kann der Spender 1 in einer mitführbaren Vorratsbox 46 aufgenommen sein, wozu Letztere ein entsprechend dem Grundriss des Spenders 1 ausgeformtes Aufnahmefach 47 aufweist. In einem dem Aufnahmefach 47 benachbarten Fach 48 ist eine Vielzahl von Blistern 18 bevorratet, in dem dargestellten Ausführungsbeispiel fünf Blister 18.

Das Fach 48 weist zur sicheren Halterung der Blister 18 Aufnahmen 49 auf, die der Aufnahme 13 im Gehäuse 2 entsprechen. In diese Aufnahmen 49 sind die Behälterteile 14 der Blister 18 eingelegt.

Die freien Enden der Blister 18 ragen zur erleichterten Entnahme derselben frei über eine in dem Fach 48 ausgeformten Vertiefung 50.

Die Vorratsbox 46 ist durch einen Deckel 51 verschließbar.

Insgesamt weist die Vorratsbox 46 eine handliche Größe auf, die es erlaubt, diese beispielsweise in einer Jackentasche mit zu tragen.

**BEZUGSZEICHENLISTE**

| | |
|---|---|
| 1 | Spender |
| 2 | Gehäuse |
| 3 | Mundstück |
| 4 | Saugkanal |
| 5 | Saugmund |
| 6 | Verschlusskappe |
| 7 | Wirbelkammer |
| 8 | Kammerdecke |
| 9 | Kammerboden |
| 10 | Gehäuseteil |
| 11 | Stufe |
| 12 | Luftkanal |
| 13 | Aufnahme |
| 14 | Behälterteil |
| 15 | Stützabschnitt |
| 16 | Behälterabdeckung |
| 17 | Abziehhandhabe |
| 18 | Blister |
| 19 | Öffnungsklappe |
| 20 | Klappendecke |
| 21 | Seitenwände |
| 22 | Vertiefung |
| 23 | Vorsprung |
| 24 | Deckenunterseite |
| 25 | Abziehöffnung |
| 26 | Rückwand |
| 27 | Sammelkanal |
| 28 | Umlenkabschnitt |
| 29 | Einlassöffnung |
| 30 | Behälterkanal |
| 31 | Direktkanal |
| 32 | Vereinigungsabschnitt |
| 33 | oberes Stockwerk |
| 34 | Ringwandung |
| 35 | Ringraum |
| 36 | Stockwerkboden |
| 37 | Öffnung |
| 38 | unterstes Stockwerk |
| 39 | Ringraum |
| 40 | Trennwand |
| 41 | Außenluftöffnung |
| 42 | Außenluftkanal |
| 43 | Markierungslinie |
| 44 | Zentralraum |
| 45 | Durchströmöffnung |
| 46 | Vorratsbox |
| 47 | Aufnahmefach |
| 48 | Fach |
| 49 | Aufnahme |
| 50 | Vertiefung |
| 51 | Deckel |
| | |
| a | Luftstrom |
| b | Luftstrom |
| c | Luftstrom |
| | |
| M | Masse |
| r | Abziehrichtung |
| x | Klappenachse |
| y | Wirbelkammer-Achse |

## Patentansprüche

1. Spender (1) für in einer gesonderten Verpackung enthaltene, pulverförmige Massen, insbesondere in einer Blisterverpackung enthaltene Massen (M), welcher Spender eine Aufnahmevertiefung (13) für ein einsenkbares Behälterteil (14) mit abziehbarer Behälterabdeckung (16) aufweist, welch letztere durch eine Abziehöffnung (25) nach außen ragt und diese in Abzugsendstellung verschließt und wobei ein quer zur Abziehrichtung (r) in den Spender (1) eingesaugter Luftstrom zu einem Mundstück (3) geführt ist, **dadurch gekennzeichnet, dass** sich die Einlassöffnungen (29) zum Eintritt des Luftstromes getrennt seitlich der Abziehöffnung (25) in einer durchsichtigen Öffnungsklappe (19) befinden, durch welche eine Markierungslinie (43) sichtbar ist als Richtwert für das Herausziehen der Behälterabdeckung (16).

2. Spender nach Anspruch 1, **dadurch gekennzeichnet, dass** ein Teil des Luftstroms nach Durchsetzen des Behälterteils (14) ebenengleich in einen Umlenkabschnitt (28) einer Wirbelkammer (7) eintritt, wobei die Wirbelkammer (7) sich über zwei Stockwerke (33, 38) erstreckt und wobei weiter in die Wirbelkammer (7) eine oder mehrere zusätzliche Außenluftöffnungen (41) münden.

3. Spender nach Anspruch 1, **dadurch gekennzeichnet, dass** die eintretende Luft zunächst einen Sammelkanal (27) durchsetzt, der sich in einen, unter Umgehung des Behälterteils (14) zu einem Saugmund (5) führenden Direktkanal (31) und weiter in einen zum Behälterteil (14) führenden Behälterkanal (30) aufspaltet.

4. Spender nach einem oder mehreren der vorhergehenden Ansprüche, **dadurch gekennzeichnet, dass** der Behälterkanal (30) und der Direktkanal (31) in der Wirbelkammer (7) münden, nachdem sie sich vor der Wirbelkammer (7) vereinigt haben.

5. Spender nach einem oder mehreren der vorhergehenden Ansprüche, **dadurch gekennzeichnet**, das der Behälterkanal (30) und/oder der Direktkanal (31) in ein oberes Stockwerk (33) der über zwei Stockwerke (33, 38) ausgebildeten Wirbelkammer (7) treten.

6. Spender nach einem oder mehreren der vorhergehenden Ansprüche, **dadurch gekennzeichnet, dass** die Öffnungsklappe (19) Ausformungen aufweist, welche in Zusammenwirkung mit dem Aufnahmebereich für das Behälterteil (14) die gewünschten Luftwege erbringen.

7. Spender nach einem oder mehreren der vorhergehenden Ansprüche, **dadurch gekennzeichnet, dass** für die eintretende Luft eine Mehrzahl von Einlassöffnungen (29) vorgesehen sind, welche nutartig ausgebildet und quer zur Längserstreckungsrichtung des Spenders ausgerichtet sind.

8. Spender nach einem oder mehreren der vorhergehenden Ansprüche, **dadurch gekennzeichnet, dass** die Einlassöffnungen (29) teilweise nach oben und teilweise seitlich geöffnet sind.

## Claims

1. Dispenser (1) for pulverulent substances contained in a separate pack, in particular substances (M) contained in a blister pack, which dispenser has a holding recess (13) for a container part (14) that can be inserted into the recess, the container part having a pull-off container covering (16), which latter projects outwardly through a pull-off opening (25) and closes this opening in the pull-off end position, and an air stream which is sucked into the dispenser (1) transversely to the pull-off direction (r) being guided to a mouthpiece (3), **characterized in that** inlet openings (29) for entry of the air stream are located, spaced apart, in a transparent opening flap (19) and to the side of the pull-off opening (25), through which flap a marking line (43) is visible as a point of reference for the pulling-out of the container covering (16).

2. Dispenser according to Claim 1, **characterized in that** a part of the air stream, following passage through the container part (14), enters, in the same plane, into a deflecting portion (28) of a vortex chamber (7), the vortex chamber (7) extending over two levels (33, 38) and, furthermore, one or more additional outside-air openings (41) opening out in the vortex chamber (7).

3. Dispenser according to Claim 1, **characterized in that** the incoming air passes, in first instance, through a collecting channel (27) which splits up into a direct channel (31) that leads to a suction mouth (5), bypassing the container part (14) in the process, and also into a container channel (30) that leads to the container part (14).

4. Dispenser according to one or more of the preceding claims, **characterized in that** the container channel (30) and the direct channel (31) open out in the vortex chamber (7) after they have combined before the vortex chamber (7).

5. Dispenser according to one or more of the preceding claims, **characterized in that** the container channel (30) and/or the direct channel (31) enter/enters into an upper level (33) of the vortex chamber (7), which is formed over two levels (33, 38).

6. Dispenser according to one or more of the preceding claims, **characterized in that** the opening flap (19) has shaped structures which, in conjunction with the holding region for the container part (14), create the desired air paths.

7. Dispenser according to one or more of the preceding claims, **characterized in that** a plurality of inlet openings (29) are provided for the incoming air, which inlet openings are formed in a groove-like manner and are oriented transversely to the longitudinal direction in which the dispenser extends.

8. Dispenser according to one or more of the preceding claims, **characterized in that** the inlet openings (29) are open, in part, in the upward direction, and, in part, laterally.

## Revendications

1. Distributeur (1) pour des substances pulvérulentes contenues dans un emballage séparé, en particulier des substances (M) contenues dans un emballage blister, distributeur qui présente un creux de réception (13) destiné à recevoir une partie de récipient insérable (14) avec un couvercle de récipient rétractable (16), ce dernier s'étendant vers l'extérieur à travers une ouverture de traction (25) et fermant celle-ci dans sa position rétractée extrême, et dans lequel un courant d'air aspiré dans le distributeur (1) transversalement à la direction de traction (r) est conduit à une embouchure (3), **caractérisé en ce que** les ouvertures d'entrée (29) destinées à l'entrée du courant d'air se trouvent séparées latéralement de l'ouverture de traction (25) dans un clapet d'ouverture transparent (19), à travers lequel une ligne de marquage (43) est visible comme valeur indicative pour l'extraction du couvercle de récipient (16).

2. Dispositif selon la revendication 1, **caractérisé en ce qu'**une partie du courant d'air pénètre, après avoir traversé la partie de récipient (14), dans un même plan dans une partie de déviation (28) d'une chambre de fluidisation (7), dans lequel la chambre de fluidisation (7) s'étend sur deux étages (33, 38) et dans lequel une ou plusieurs ouvertures d'air extérieur supplémentaires (41) débouchent en plus dans la chambre de fluidisation (7).

3. Distributeur selon la revendication 1, **caractérisé en ce que** l'air entrant traverse d'abord un canal de collecte (27), qui se divise en un canal direct (31) menant à une bouche d'aspiration (5) en contournant la partie de récipient (14) et en plus en un canal de récipient (30) menant à la partie de récipient (14).

4. Distributeur selon une ou plusieurs des revendications précédentes, **caractérisé en ce que** le canal de récipient (30) et le canal direct (31) débouchent dans la chambre de fluidisation (7), après qu'ils se soient réunis avant la chambre de fluidisation (7).

5. Distributeur selon une ou plusieurs des revendications précédentes, **caractérisé en ce que** le canal de récipient (30) et/ou le canal direct (31) arrivent dans un étage supérieur (33) de la chambre de fluidisation (7) formée sur deux étages (33, 38).

6. Distributeur selon une ou plusieurs des revendications précédentes, **caractérisé en ce que** le clapet d'ouverture (19) présente des déformations qui créent les chemins d'air désirés en coopération avec la zone de réception destinée à la partie de récipient (14).

7. Distributeur selon une ou plusieurs des revendications précédentes, **caractérisé en ce qu'**il est prévu pour l'air entrant une pluralité d'ouvertures d'entrée (29), qui sont réalisées en forme de rainures et qui sont orientées transversalement à la direction d'extension longitudinale du distributeur.

8. Distributeur selon une ou plusieurs des revendications précédentes, **caractérisé en ce que** les ouvertures d'entrée (29) sont ouvertes en partie vers le haut et en partie latéralement.
